# EUROPÄISCHE PATENTANMELDUNG

(11) **EP 0 697 392 A1**
(43) Veröffentlichungstag der Anmeldung: **21.02.1996**
(21) Anmeldenummer: 95112479.1
(22) Anmeldetag: 09.08.1995
(51) Int. Cl.: C07C 45/60, C07C 47/12

(54) **Verfahren zur Herstellung von Glutardialdehyd**

(30) Priorität: 18.08.1994 DE 4429262
(71) Anmelder: BASF AKTIENGESELLSCHAFT, D-67056 Ludwigshafen (DE)
(72) Erfinder: Becker, Rainer, Dr., D-67098 Bad Dürkheim (DE); Friedrich, Wolfgang, D-67346 Speyer (DE); Klink, Walter, Dr., D-67134 Birkenheide (DE); Schossig, Jürgen, Dr., D-67136 Fussgönheim (DE); Henne, Andreas, Dr., D-67433 Neustadt (DE)

(57) **Zusammenfassung**

Verfahren zur Herstellung von Glutardialdehyd durch Umsetzung von Alkoxidihydropyranen der allgemeinen Formel I 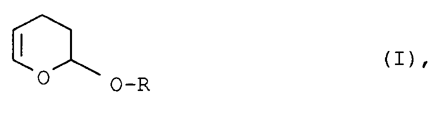
in der R für C₁- bis C₂₀-Alkoxy steht, mit Wasser bei Temperaturen von 0 bis 150°C und Drücken von 0,01 bis 50 bar in Gegenwart eines Katalysators, indem man als Katalysator Bleicherden einsetzt.

## Beschreibung

Die vorliegende Erfindung betrifft ein Verfahren zur Herstellung von Glutardialdehyd durch Umsetzung von Alkoxydihydropyranen mit Wasser in Gegenwart von Bleicherden als Katalysator.

Aus der US-A-2,546,018 ist die thermische Hydrolyse von Alkoxydihydropyranen bei Temperaturen von 100 bis 200°C zu Glutar-dialdehyd bekannt. Zur Erniedrigung der Reaktionstemperatur werden saure Katalysatoren vorgeschlagen.

Homogene Säurekatalyse mit z.B. H₃PO₄ ist aus JP 72 26 488 bekannt. Die hier beschriebenen Verwendungen von homogenen Säuren als Katalysatoren für die Hydrolyse des Alkoxypyrans zu Glutardialdehyd haben den Nachteil, daß sie nach beendeter Umsetzung im System verbleiben und daher neutralisiert werden müssen. In dieser Form verursachen sie Verfärbungen des Glutardialdehyds bzw. sie katalysieren die Polymerisation des Glutardialdehyds und bewirken damit unerwünschte Trübungen durch Polymerabscheidungen. Die Polymerisationsneigung des Glutardialdehyds ist bekannt und wird im allgemeinen durch Handhabung in wäßrig-verdünnter Lösung weitgehend unterdrückt. Aufgrund dieser Problematik wurden verschiedene Verfahren unter anderem unter Verwendung von sauren Ionentauschern als Hydrolysekatalysatoren beschrieben (EP-A-66 224, US-A-4 244 876 und US-A-4 448 977). Dort wird die Umsetzung von Alkoxydihydropyran mit Wasser oder Alkohol zu Hydroxyalkoxytetrahydropyran oder zu Dialkoxytetrahydropyran als stabile Glutardialdehyd-Vorstufe beschrieben. Die Umsetzung zum gewünschten Glutardialdehyd muß also anschließend noch durchgeführt werden. Neben diesem Nachteil sind hier nicht ausreichende Katalysatoraktivität bzw. -standzeit sowie der hohe Preis der Ionentauscher unbefriedigend.

Der vorliegenden Erfindung lag daher die Aufgabe zugrunde, den zuvor genannten Nachteilen abzuhelfen.

Demgemäß wurde ein neues und verbessertes Verfahren zur Herstellung von Glutardialdehyd durch Umsetzung von Alkoxydihydropyranen der allgemeinen Formel I 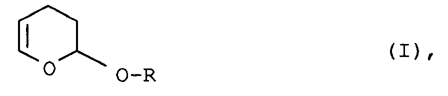
in der R für C₁- bis C₂₀-Alkoxy steht, mit Wasser bei Temperaturen von 0 bis 150°C und Drücken von 0,01 bis 50 bar in Gegenwart eines Katalysators gefunden, welches dadurch gekennzeichnet ist, daß man als Katalysator Bleicherden einsetzt.

Das erfindungsgemäße Verfahren kann wie folgt durchgeführt werden:
Das Alkoxidihydropyran I und Wasser können bei Temperaturen von 0 bis 150°C, bevorzugt 30 bis 100°C, besonders bevorzugt 40 bis 80°C und Drücken von 0,01 bis 50 bar, bevorzugt 0,1 bis 5 bar, besonders bevorzugt bei Atmosphärendruck (Normaldruck) in Gegenwart von Bleicherden als Katalysator zum Glutardialdehyd umgesetzt werden.

Als Bleicherden-Katalysatoren eignen sich beispielsweise Aluminiumhydrosilikate oder Aluminium-Magnesium-Hydrosilikate vom Typ des Montmorillonits, die z.B. durch Säuren aktiviert sein können und beispielsweise unter dem Namen "Tonsil" im Handel (z.B. Fa. Südchemie) erhältlich sind. Bleicherden werden in der Industrie bekanntlich unter anderem als saure Katalysatoren bei der Alkylierung von Phenolen oder aromatischen Aminen verwendet. Ihre katalytische Wirkung bei der Herstellung von Glutardialdehyd war bislang unbekannt.

Aus dem oben genannten Stand der Technik ergibt sich, daß als Zwischenstufen 2-Hydroxy-6-alkoxytetrahydropyrane durchlaufen werden. Diese Zwischenstufen lassen sich - abhängig vom Wasserüberschuß - analytisch nachweisen oder auch isolieren.

Das Molverhältnis von Wasser zum Alkoxidihydropyran I beträgt in der Regel 1:1 bis 100:1, bevorzugt 1:1 bis 20:1, besonders bevorzugt 1:1 bis 10:1.

Der Substituent R in den Verbindungen I hat folgende Bedeutungen:
R
- C₁- bis C₂₀-Alkoxy, bevorzugt C₁- bis C₈-Alkoxy, besonders bevorzugt C₁- bis C₄-Alkoxy wie Methoxy, Ethoxy, n-Propoxy, iso-Propoxy, n-Butoxy, iso-Butoxy, sec.-Butoxy oder tert.-Butoxy, insbesondere Methoxy oder Ethoxy.

Als Alkoxidihydropyrane I eignen sich bevorzugt 2-Alkoxy-3,4-dihydropyrane wie 2-Methoxy-3,4-dihydropyran, 2-Ethoxy-3,4-dihydropyran, 2-n-Propoxy-3,4-dihydropyran, 2-iso-Propoxy-3,4-dihydropyran, 2-n-Butoxy-3,4-dihydropyran, 2-iso-Butoxy-3,4-dihydrorohrpyran, 2-sec.-Butoxy-3,4-dihydropyran und 2-tert.-Butoxy-3,4-dihydropyran, besonders bevorzugt 2-Methoxy-3,4-dihydropyran und 2-Ethoxy-3,4-dihydropyran.

Glutardialdehyd wird z.B. in der Gerberei oder als Mikrobiozid verwendet.

### Beispiele

### Beispiel 1

### Diskontinuierliche Fahrweise

In einem 3-Halsrührkolben werden 114 g Methoxydihydropyran (1 Mol) mit 57 g Wasser (3,2 Mol) und 5 g Katalysator KSF (bezogen von Fa. Südchemie) bei 50°C gerührt. Der Umsatz betragt nach 90 min etwa 70 %, nach 5 Std. > 99 % (gaschromatographische Kontrolle). Nach 5 Std. wird abgekühlt und vom Katalysator abfiltriert, das Rohgemisch enthält nach GC < 0,1 % Ausgangsprodukt und 73 % Glutardialdehyd. Nach Abdestillieren des abgespaltenen Methanols liegt der Glutardialdehydgehalt > 90 % (ber. wasserfrei).

### Beispiel 2

### Kontinuierliche Fahrweise

In einem Rohrreaktor, gefüllt mit 1,2 l Katalysatorsträngen (K10 der Fa. Südchemie) wird durch kontinuierliches Zufahren von 40 ml/h Methoxydihydropyran (0,36 Mol) und 20 ml/h Wasser (1,1 Mol) bei 70°C und Umpumpen von 12 l/h Reaktionslösung Methoxydihydropyran zu Glutardialdehyd hydrolysiert. Der Reaktoraustrag ist einphasig und klar, die Zusammensetzung laut GC beträgt über eine Laufzeit von 20 Tagen unverändert - nach Abdestillieren des Methanols - < 1 % Methoxydihydropyran und 85-90 % Glutardialdehyd.

## Patentansprüche

1. Verfahren zur Herstellung von Glutardialdehyd durch Umsetzung von Alkoxydihydropyranen der allgemeinen Formel I 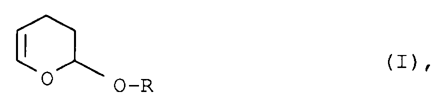 in der R für C₁- bis C₂₀-Alkoxy steht, mit Wasser bei Temperaturen von 0 bis 150°C und Drücken von 0,01 bis 50 bar in Gegenwart eines Katalysators, dadurch gekennzeichnet, daß man als Katalysator Bleicherden einsetzt.

2. Verfahren zur Herstellung von Glutardialdehyd nach Anspruch 1, dadurch gekennzeichnet, daß R für C₁- bis C₈-Alkoxy steht.

3. Verfahren zur Herstellung von Glutardialdehyd nach Anspruch 1, dadurch gekennzeichnet, daß R für C₁- bis C₄-Alkoxy steht.

4. Verfahren zur Herstellung von Glutardialdehyd nach Anspruch 1, dadurch gekennzeichnet, daß R für Methoxy oder Ethoxy steht.

5. Verfahren zur Herstellung von Glutardialdehyd nach Anspruch 1, dadurch gekennzeichnet, daß man die Umsetzung bei Temperaturen von 30 bis 100°C durchführt.

6. Verfahren zur Herstellung von Glutardialdehyd nach Anspruch 1, dadurch gekennzeichnet, daß man die Umsetzung bei Drücken von 0,1 bis 5 bar durchführt.

7. Verfahren zur Herstellung von Glutardialdehyd nach Anspruch 1, dadurch gekennzeichnet, daß man die Umsetzung bei Atmosphärendruck durchführt.
